# EUROPEAN PATENT APPLICATION

(11) **EP 2 476 419 A1**
(43) Date of publication of application: **18.07.2012**
(21) Application number: 11827038.8
(22) Date of filing: 11.05.2011
(51) Int. Cl.: A61K 31/665, A61K 9/51, A61K 47/04, A61P 31/04

(54) **PHARMACEUTICAL COMPOSITION HAVING ANTIMICROBIAL AND ANTI-INFLAMMATORY ACTIVITY FOR PARENTERAL ADMINISTRATION, PROCESS FOR PREPARING SAME**

(30) Priority: 20.09.2010 EA 201001507
(71) Applicant: Limonov, Viktor Lvovich, Moscow 121374 (RU)
(72) Inventor: GAIDUL, Konstantin Valentinovich, Novosibirsk 630089 (RU); DUSHKIN, Aleksandr Valerevich, Novosibirsk 630117 (RU)
(74) Representative: von Füner, Nicolai
(86) International application number: PCT/RU2011/000321
(87) International publication number: WO 2012/039641

(57) **Abstract**

The present invention is related to pharmacology, medicine, veterinary science and pharmaceutical industry, in particular to the process of production original composite antimicrobial and anti-inflammatory preparations for parenteral administration having an increased therapeutic efficiency in the treatment of severe forms of infectious inflammatory diseases.

The claimed pharmaceutical composition contains fosfomycin and finely dispersed nanostructured silica dioxide active agents. The claimed process for the production of a pharmaceutical composition includes mixing fosfomycin with finely dispersed nanostructured silica dioxide, characterized in that the mixture of said substances in a weight ratio from 10:1 to 70:1 respectively is being exposed to mechanical treatment by impact and abrasive actions to increase the weight percentage of the finely dispersed fraction (≤ 5 micron) to at least 25%. The received mixture is used to prepare for injection solutions.

## Description

This invention relates to antimicrobial pharmaceutical preparations and technologies for the production thereof. It can be used in medicine and veterinary science for treating inflammatory infectious diseases, as well as in pharmaceutical industry for the manufacture of medicaments.

At the moment, to ensure a successful therapy of many inflammatory infectious diseases (sepsis, meningitis, osteomyelitis, pyelonephritis and etc.) practicing physicians of different specializations are widely using an antibiotic with the international nonproprietary name - fosfomycin [1, 2, 3, 4, 5].

The mentioned antibiotic possesses a wide range of antimicrobial action and provides a bactericidal effect on many gram-positive and gram-negative microorganisms which provoke infectious processes in different organs and tissues.

The secondary resistance of microorganisms towards fosfomycin develops slowly, and the absence of cross-resistance with other antibiotics allows successfully combining it with betalactam and aminoglycosides in order to achieve a clear synergism [1, 6, 7].

Fosfomycin has a unique ability to inhibit the synthesis and proinflammatory cytokines (IL-1, IL-6, TNF and etc.) with human blood cells, which has a high clinical significance during generalized infections treatment, in particular sepsis [8].

However, when taking into consideration all positive properties of fosformycin it is necessary to emphasise that a monotherapy with this medicament of inflammatory infectious processes provoked by microorganisms having different levels of resistance to this antibiotic, is not always efficient. This fact forces many specialists to replace fosfomycin by other antibiotics or inject them simultaneously with the other antimicrobial agents [6, 9], which is not advisable from clinical and economical point of view.

Therefore, the development of new approaches in order to increase the antimicrobial activity and clinical effectiveness of fosfomycin is a relevant objective of the modem experimental pharmacology and practical medicine.

In the last few years it has been discovered that using different nano-particles as delivering agents for delivering different antibiotics into bacteria and macrophages to increase their concentration at the inflammation area and consequently improve their antimicrobial properties, as well as to stimulate the antibacterial activity of macrophages and their additional recruitment into the infected tissues, is a very prospective tendency for the development of original technologies and methods of antibiotic therapy [10, 11, 12, 13, 14, 15, 16, 17, 18, 19].

The essence of this invention consists in that it is suggested to use SiO₂ (silica dioxide) nano-particles which are capable of serving as endocellular delivery agents to deliver antibiotics into macrophages in case of parenteral administration due to the pharmacologically effective qualities of biocompatibility, biodistribution, biodegradation and low toxicity of said nano-particles (regardless of the level of structure porosity). The macrophages are concentrated in the areas of inflammation which can be observed in lungs, liver, kidneys, lien, lymph glands, heart, skin, urinary bladder and other organs of the mammals (i.e. the antibiotic concentration is considerably increased in the infected areas). Said nano-particles can also stimulate the antimicrobial activity of the afore-mentioned immune system cells what leads to a significant increase of the therapeutic effect of antimicrobial preparations in the treatment of inflammatory infectious diseases treatment [20, 21, 22, 23, 24, 25, 26, 27].

The present invention solves the problem of producing an antimicrobial and anti-inflammatory pharmaceutical composition for injections based on the use of fosfomycin and silica dioxide nano-particles having an increases therapeutic activity (compared to the conventional fosfomycin which is considered as the prototype of the present invention) in the treatment of inflammatory infectious diseases.

To problem of the present invention can be solved by using an antimicrobial and anti-inflammatory pharmaceutical composition for parenteral administration which contains antibiotic fosfomycin as a therapeutic agent in form of injection powder as well as finely dispersed nanostructured silica dioxide having a weight ratio fosfomycin : finely dispersed nanostructured silica dioxide of (10-70) : 1.

The percentage of the finely dispersed nanostructured silica dioxide having a size of ≤ 5 micron is at least 25%.

In order to solve the problem of the present invention it is also suggested to use an antimicrobial and anti-inflammatory pharmaceutical composition for parenteral administration produced by mixing fosfomycin with other components, i.e. mixing fosfomycin injection powder with finely dispersed nanostructured silica dioxide in a weight ratio fosfomycin : finely dispersed nanostructured silica dioxide of (10-70) : 1. The obtained mixture is subjected to mechanical treatment by impact and abrasive actions.

The obtained mixture is subjected to mechanical treatment by impact and abrasive actions in order to obtain at least 25% of the finely dispersed nanostructured silica dioxide having a size of ≤ 5 micron.

Therapeutic efficiency of the proposed pharmaceutical composition will increase if it the obtained mixture is subjected to mechanical treatment by impact and abrasive actions in order to obtain at least 25% of the finely dispersed nanostructured silica dioxide having a size of ≤ 5 micron.

To prepare the mentioned pharmaceutical composition fosfomycin (parenteral administration form) was used produced by a Spanish company "Ercros". As a finely dispersed nanostructured silica dioxide (hereafter referred to as BHSiO₂) "Polysorb" drug was used (pharmacological group: enterosorbing solution; active substance: colloidal silica dioxide) produced by Russian company CJSC "Polysorb", containing round shaped silica dioxide nanoparticles (dimension 5-20 nm) combined into aggregates (irregular microparticles) with a size of ≤ 90 micron (registration number Nº 001140101-100908). Similar preparation is produced by Ukrainian company CJSC "Biopharma" under a trade name "Silics" [24].

The selection of the make-up of the formulation was based on the phenomenon of reversible sorption of fosfomycin molecules by and nano- as well as micro particles of BHSiO₂, as well as the reduction of BHSiO₂ particles during the mechanical activation of mixtures thereof with fosfomycin substances by an intensive impact and abrasive mechanical action.

The claimed process for the production of the afore-mentioned pharmaceutical composition by activating the fosfomycin and BHSiO₂ powder mixture with an intensive impact and abrasive action allows increasing the percentage of finely dispersed BHSiO₂ particles (≤ 5 micron) on which fosfomycin molecules are adsorbed and which are phagocyted by mostly macrophages [28].

To achieve this goal the mixture of the above-mentioned materials in a weight ratio fosfomycin : BHSiO₂ of (10-75) : 1 is exposed to intensive impact and abrasive mechanical activation until the percentage of the finely dispersed fraction is increased to at least 25%.

From the obtained powder composition you can prepare an injection colloidal solutions for parenteral administration (it is dissolved by any process appropriate for fosfomycin) consisting of finely dispersed BHSiO₂ particles and fosfomycin molecules reversibly sorbed on its surface.

Introducing of the finely dispersed nanostructured silica dioxide in the weight ratio fosfomycin : BHSi0₂ of 10:1 to 75:1 is determined by the combination of 2 factors: 1) if the content of BHSiO₂ is increased to more than 10% based on the weight of the total composition, laboratory animals suffer from the blockage of small capillary tubes in solid viscus; 2) if the BHSiO₂ content is decreased to more than 14% based on the weight of the composition its therapeutic efficiency (in particular during the treatment of bacterial sepsis in mice) doesn't significantly differ from the basic efficiency if the initial antibiotic.

For the production of the composition a mechanochemical method was used consisting in the treatment of mixture of solid components by intensive mechanical impacts - pressure and shearing, mostly carried out in different kind of mills which perform impact and abrasive actions on the substances. The mixture of the solid fosfomycin substance and finely dispersed nanostructured silica dioxide taken in the ratio from 10:1 to 75:1 1 by weight is exposed to mechanical activation in bead mills. The used method for the production of mixtures significantly helps to avoid chemical degradation of the antibiotic and achieve full homogeneity of powder components in comparison with producing the mixture by simple mixing of components or evaporating solutions thereof. As a result a high pharmacological activity of pharmaceutical composition is ensured.

As a quantitative criterion of the minimum necessary mechanical impact it is suitable to use the method of granulometry for the suspension of the obtained compositions. It is necessary that the percentage by weight of the particles having a size of smaller than 5 micron is at least 25%. The mechanical treatment of powder mixtures is performed in rotary, vibrational and planetary mills. As grinding bodies, balls, cores and etc. can be used.

Pharmacological tests of the compositions with laboratory animals (mice) have shown, that the mentioned compositions prepared by the mentioned method have a higher therapeutic efficiency in the treatment of bacterial sepsis, provoked by *Staphylococcus aureus, Escherichia coli* and *Pseudomonas aeruginosa,* comparing to the initial fosfomycin.

Consequntly, using the mentioned pharmaceutical compositions and the process for production thereof provides the following advantages:
1) Clinically significant increase of the effectiveness and quality of the antimicrobial therapy of severe inflammatory infectious diseases, death rate reduction;
2) Ecological safety, lack of wastes and low price of pharmaceutical production technology.

The offered invention is illustrated by examples listed below.

### Example Nº1. Solid composition production: fosfomycin/ BHSiO₂.

The mixture of fosfomycin and BHSiO₂ in weight ratio 10:1, 30:1 and spa:1 1 is being processed for 1, 2 or 4 hours in a rotary mill. In table Nº1 the data of the granulometric make-up of the water suspensions of the obtained composition variants are indicated (Micro-Sizer 201 laser granulometer was used) as well as results of the HELC analysis which show the content of antibiotics in them (in % from the initial substance) and fosfomycin sorption degree (in %) by BHSiO₂ particles.

As you can see from table Nº1 the chosen conditions for the production of the composition allow increasing the percentage of the finely dispersed BHSiO₂ fraction up to a certain value (not less than 25% from the total weight) (particles size less than 5 micron) and avoiding the chemical degradation of the antibiotic.

**Table Nº1**

| **Granulometric make-up of water suspensions, fosfomycin sorption rate and content in different variants of the composition** | | | | |
|---|---|---|---|---|
| Make-up of the composition | Size and content % of BHSiO₂ particles* | | Fosfomycin sorption rate by BHSiO₂ particles (%) | Fosfomycin content (%) |
| | % < 3 micron | %<5 micron | | |
| Initial BHSiO₂ | 0,5 | 5,7 | - | - |
| Fosfomycin:BHSiO₂ (10:1), m/a 1 hour | 12,3 | 28,9 | 30,2 | 99 |
| Fosfomycin:BHSiO₂(30:1), m/a 2 hours | 17,6 | 31,8 | 41,1 | 99 |
| Fosfomycin:BHSiO₂ (50:1), m/a 4 hours | 16,1 | 30,7 | 42,9 | 98 |

| | | | | |
|---|---|---|---|---|
| * - finely dispersed nanostructured silica dioxide | | | | |

### Example Nº2. Determination of the therapeutic efficiency of fosfomycin and pharmaceutical compositions.

There has been a research of fosfomycin mechanized for 2 hours and composed of a mixture antibiotic/ BHSiO₂ in the weight ratio 30:1 and 50:1 respectively.

To determine therapeutic efficiency of fosfomycin and pharmaceutical compositions including BHSiO₂, we used experimental sepsis models and a statistical processing methods of the received data (χ²) according to [29, 30] .

Microorganisms: *Staphylococcus aureus* (ATCC Nº 25923 F-49), *Escherichia coli* (ATCC Nº25922 F-50), *Pseudomonas aeruginosa* (ATCC Nº27853 F-51).

Animals: for the experiments we used hybrid mice (CBA x C₅₇Black/₆)CBF₁ according to the "Regulations for test animals use" (USSR Ministry of health order supplement Nº755 from 12.08. 1977).

### Experimental sepsis models

The mice have been injected 0,8ml of a suspension of *Pseudomonas aeruginosa* daily culture in an amount of 5x10⁸ CFU/mouse or a suspension *of Staphylococcus aureus* daily culture in an amount of 10¹⁰ CFU/mouse or a suspension of *Escherichia coli* daily culture in an amount of 8x10⁸ CFU/mouse. The control group has been injected 0,8ml of physiological solution (0,9% sodium chloride solution). In a day after being infected the test mice have been daily (during 3 days) intravenous injected with 100mg/kg of antibiotics or different pharmaceutical compositions (antibiotic/ BHSiO₂ watered down with 0,25ml of physiological solution. The control group of mice has been injected 0,25mg of a physiological solution using the same scheme.

Antimicrobial therapy efficiency has been estimated basing on the number of the living mice on the 7^{th} day of being infected [29, 30].

The received data indicated in table Nº2 reflect the results of 3 independent experiments conducted for each test group.

**Table Nº2**

| **Thepapeutic efficiency of the antimicrobial therapy of bacterial sepsis** | | | |
|---|---|---|---|
| Test groups | Mice survival rate on the 7^{th} day of infection* | | |
| | *Staphylococcus aureus* | *Escherichia coli* | *Pseudomonas aeruginosa* |
| Physiological solution (control) | 0% (0/30) | 0% (0/31) | 0% (0/30) |
| Fosfomycin | 40% (12/30) | 46,6% (14/30) | 32,2% (10/31) |
| Fosfomycin/BHSiO₂ (30:1), | 83,3% (25/30) | 87% (27/31) | 76,6% (23/30) |
| m/a 2 hours | P<0,01** | P<0,01** | P<0,01** |
| Fosfomycin/BHSiO₂ (50: 1), m/a 2 hours | 80,6% (25/31) | 87,5% (28/32) | 75,7% (25/33) |
| | P<0,01** | P<0,01** | P<0,01** |

| | | | |
|---|---|---|---|
| *- survival rate/infected animals rate measured in % and absolute values **- comparing to the group of mice having Fosfomycin | | | |

As you may see in Table Nº2 the suggested pharmaceutical composition (fosfomycin/BHSiO₂) reliably possess an increased therapeutic efficiency (2 times higher) comparing to simple fosfomycin in case of lab animals sepsis treatment, provoked by *Pseudomonas aeruginosa, Staphylococcus aureus* or *Escherichia coli.*

**Example Nº3**. Determining the anti-inflammatory action of fosfomycin and pharmaceutical compositionh.

It is known that in case of sepsis the quantity of leucocytes in the peripheral blood can reflects the activity of the inflammatory process [31].

We have studied the leukocytes quantity the peripheral blood of mice that have survived until the day 7 after sepsis induction according to the method described above. The received data is shown in the table Nº3.

**Table Nº3**

| **Peripheral blood leukocytes quantity of the mice which have survived until day 7 after being infected with *Pseudomonas aeruginosa* and *Staphylococcus aureus* treated with fosfomycin and pharmaceutical composition** | |
|---|---|
| Test groups (each group contains not less than 10 mice) | Leukocytes quantity x 10⁹ /l Me(LQ-HQ)* |
| 1. Intact mice. Physiological solution (control) | 8,31 (6,55-9,44) |
| 2. Mice infected with *Staphylococcus aureus.* | 17,38 (15,5-21,5) |
| Fosfomycin | P₁₋₂<0,001 |
| 3. Mice infected with *Staphylococcus aureus.* | 12,25 (11,63-13,75) |
| | P₁₋₃<0,01 |
| Fosfomycin:BHSiO₂ (30:1), m/a 2 hours | P₂₋₃<0,05 |
| 4. Mice infected with *Pseudomonas aeruginosa.* | 17,13 (16,0-18,63) |
| Fosfomycin | P₁₋₄<0.001 |
| 5. Mice infected with *Pseudomonas aeruginosa.* | 12,14 (11,53-13,0) |
| | P₁₋₅<0,01 |
| Fosfomycin:BHSiO₂ (30:1), m/a 2 hours | P₄₋₅<0,02 |

| | |
|---|---|
| *- median, low and high quartiles | |

As you may see from Table Nº3 the proposed pharmaceutical composition (fosfomycin/BHSiO₂) has a reliably expressed anti-inflammatory action compared with fosfomycin in case of treatment of sepsis provoked by *Pseudomonas aeruginosa* or *Staphylococcus aureus* in test animals.

In that case, on the basis of the received data we may come to the conclusion that the proposed antimicrobial and anti-inflammatory pharmaceutical composition for parenteral administration(fosfomycin/ BHSiO₂) has a significantly increased therapeutic effect in case of treating severe infectious inflammatory diseases comparing to the initial fosfomycin (prototype of the mentioned invention).

### Used literature

1. I.B. Mikhailov // Physician's handbook for clinical pharmacology. - St. Petersburg: «Foliant» Publishing, 2001. - 736 p.
2. Joukhadar C., Klein N., Dittrich P. et al. Target site penetration of fosfomycin in critically ill patients // J. Antimicrob. Chemother. - 2003. - Vol.51. - P.1247-1252.
3. Sauermann R., Karch R., Langenberger H. et al. Antibiotic abscess penetration: fosfomycin levels measured in pus and simulated concentration-time profiles // Antimicrob. Agents Chemother. - 2005. - Vol.49. - P. 4448-4454.
4. Schintler M.V., Traunmuller F., Metzler J. et al. High fosfomycin concentrations in bone and peripheral soft tissue in diabetic patients presenting with bacterial foot infection // J. Antimicrob. Chemother. - 2009. - Vol.64. - P.574-578.
5. Matzi V., Lindenmann J., Porubsky C. et al. Extracellular concentrations of fosfomycin in lung tissue of septic patients // J. Antimicrob. Chemother. - 2010. - Viol.65. - P. 995-998.
6. Kastoris A.C., Rafailidis P.I., Vouloumanou E.K. et al. Synergy of fosfomycin with other antibiotics for Gram-positive and Gram-negative bacteria // Europ. J. Clinical Pharmacology. - 2010. - Vol.66. - P.359-368.
7. Cai Y., Fan Y., Wang R. et al. Synergistic effects of aminoglicosides and fosfomycin on Pseudomonas aeruginosa in vitro and biofilm infections in a rat model // J. Antimicrob. Chemother. - 2009. - Vol.64. - P. 563-566.
8. Zeitlinger M., Marsik C., Steiner I. et al. Immunomodulatory effects of fosfomycin in an endotoxin model in human blood // J. Antimicrob. Chemother. - 2006. - Vol.59. - P. 219-223.
9. Presterl E., Hajdu S., Lassnigg A. M. et al. Effects of azithromycin in combination with vancomycin, daptomycin, fosfomycin, tigecycline and ceftriaxone on Staphylococcus epidermidis biofilms // Atimicrob. Agents Chtmother. - 2009. - Vol.53. - P. 3205-3210.
10. Abeylath S.C., Turos E. Drug delivery approaches to overcome bacterial resistance to β-lactam antibiotics // Expert Opinion on Drug Delivery. - 2008. - Vol.5. - P.931-949.
11. Bastus N.G., Sanchez-Tillo E., Pujals S. et al. Peptides conjugated to gold nanopar-ticles induce macrophage activation // Molecular Immunology. - 2009. - Vol.46. - P.743-748.
12. Pinto-Alphandary H., Andremont A., Couvreur P. Targeted delivery of antibiotics using liposomes and nanoparticles: research and applications // International Journal of Antimicrobial Agents. - 2000. - Vol. 3. - P.155-168.
13. Ulbrich W., Lamprech A. Targeted drug-delivery approaches by nanoparticulate carriers in the therapy of inflammatory diseases // Journal Royal Society Interface. - 2010. - Vol.7, Suppl. 1. - P.S55-566.
14. A.E. Guliaev, B.A. Ermekbaeva, G.Y. Kivman and etc. Nano-particles as antibiotics direct transportation vector (review) // Chenical and pharmaceutical magazine. - 1998. - Nº3.-P.3-6.
15. Rosemary M.J., MacLaren I., Pradeep T. Investigation of antibacterial properties of ciprofloxacin@SiO2. // Langmuir. - 2006. - Vol.22. - P. 10 125-10129.
16. Rai A., Prabhune A., Perry C.C. Antibiotic mediated synthesis of gold nanoparticles with potent antimicrobial activity and their application in antimicrobial coatings // Journal of Materials Chemistry. - 2010. - Vol.20. - P.6789-6798.
17. Zolnik B.S., Gonzalez-Fernandez A., Sadrieh N., Dobrovolskaia V. Minireview: Nanoparticles and the immune system // Endocrinology. - 2010. - Vol.151. - P.458-465.
18. Piatayev N.A., Beliyev A. N, Romanov M.D., Kotlov I.S. // Targetet cell-associated delivery of medicaments. - Saransk: Mordovian University Press, 2007. - 140 p.
19. Pinto-Alphandary H., Balland O., Laurent M. et al. Intracellular visualization of ampicillin-loaded nanoparticles in peritoneal macrophages infected in vitro with Salmonella typhimurium // Pharmaceutical Research. - 1994. - Vol.11. - P.38-46.
20. Park J-H., Gu L., Maltzahn G. et al. Biodegradable luminescent porous silica nanoparticles for in vivo applications // Nature Materials. - 2009. - Vol.8. - P.331-336.
21. Pernis B. Silica and the immune system // Acta Biomed. - 2005. - Vol.76, Suppl. 2.- P.38-44.
22. Tasciotti E., Liu X., Bhavane R. Et et al. Mesoporous silica particles as a multistage delivery system for imaging and therapeutic applications // Nature Nanotechnology. - 2008.-Vol.3. - P.151-157.
23. Seleem M.N., Munusamy P., Ranjan A et al. Silica-antibiotic hybrid nanoparticles for targeting intracellular pathogens // Antimicrob. Agents Chemother. - 2009. - Vol.53.-P.4270-4274.
24. Clinical chemistry and silica dioxide clinical use // Ed. by A.A. Chuyko. - Kyiv: "Naukova Dumka" Publishing, 2003. - 416 p.
25. Chuiko A., Pentyuk A., Shtat'ko E., Chuiko N. Medical aspects of application of highly disperse amorphous silica // Surface Chemistry in Biomedical and Environmental Science. Edited by J.P.Blitz and V. Gun'ko.II. Mathematics, Physics and Chemistry. - 2006.-Vol.228. - P.191-204.
26. Waters K. M., Masiello L.M., Zangar R.C. et al. Macrophage responses to silica nanoparticles are highly conserved across particle sizes // Toxicological Sciences. - 2009.-Vol.107. - P. 553-569.
27. Lucarelli M., Gatti A.M., Savarino G. et al, Innate defence functions of macrophages can be biased by nano-sized ceramic and metallic particles // European Cytokine Network.-2004. - Vol.15. - P.339-346.
28. Hamilton R.F., Thakur S.A., Mayfair J.K., Holian A. MARCO mediates silica uptake and toxicity in alveolar macrophages from C57BL/6 mice // J. Biological Chemistry. - 2006.-Vol.281. - P. 34218-34226.
29. Eckhardt C., Fickweiler K., Schaumann R. et al. Therapeutic efficacy of moxifloxacin in a murine model of severe systemic mixed infection with E.coli and B.fragilis // Anaerobe. - 2003. - Vol.9. - P.157-160.
30. Schaumann R., Blatz R., Beer J. et al. Effect of moxifloxacin versus imipenem/cilastatin treatment on the mortality of mice infected intravenously with different strains of Bacteroides fragilis and Escherichia coli // Journal of Antimicrobial Chemotherapy. - 2004. - Vol.53. - P.318-324.
31. Aird W.C. The hematologic system as a marker of organ dysfunction in sepsis // Mayo Clin. Proc. - 2003. - Vol.78. - P.869-881.

## Claims

1. Antimicrobial and anti-inflammatory pharmaceutical composition for parenteral administration containing fosfomycin as therapeutic agent, **characterized in that** it is prepared in form of a powder for preparing injection solutions and contains finely dispersed nanostructured silica dioxide in weight ratio fosfomycin : finely dispersed nanostructured silica dioxide of (10-70):1.

2. The composition according to claim 1 **characterized in that** the percentage of the finely dispersed nanostructured silica dioxide particles having a size of ≤ 5 micron is at least 25%.

3. The process for the production of the antimicrobial and anti-inflammatory pharmaceutical composition for parenteral administration comprising mixing fosfomycin with other components **characterized in that** the fosfomycin in form of a powder is mixed with powder-like finely dispersed nanostructured silica dioxide in weight ratio fosfomycin : finely dispersed nanostructured silica dioxide of (10-70):1, and the obtained mixture is subjected to a mechanical treatment by impact and abrasive actions.

4. The process according to claim 3, **characterized in that** the obtained mixture is subjected to a mechanical treatment by impact and abrasive actions so that the percentage of the finely dispersed nanostructured silica dioxide particles having a size of ≤ 5 is at least 25%.
